(19) **Europäisches Patentamt · European Patent Office · Office européen des brevets**

(11) **EP 2 967 477 B1**

(12) **EUROPEAN PATENT SPECIFICATION**

(45) Date of publication and mention of the grant of the patent:
**15.05.2019 Bulletin 2019/20**

(21) Application number: **14765037.8**

(22) Date of filing: **13.03.2014**

(51) Int Cl.:
**A61B 6/03** (2006.01)

(86) International application number:
**PCT/IL2014/050268**

(87) International publication number:
**WO 2014/141268 (18.09.2014 Gazette 2014/38)**

(54) **MICROWAVE IMAGING RESILIENT TO BACKGROUND AND SKIN CLUTTER**

GEGENÜBER HINTERGRUND UND HAUTSTÖRUNGEN ROBUSTE MIKROWELLENBILDGEBUNG

IMAGERIE PAR MICRO-ONDES RÉSISTANTE AU FOUILLIS D'ÉCHOS DE FOND ET DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2013 US 201361781314 P**

(43) Date of publication of application:
**20.01.2016 Bulletin 2016/03**

(73) Proprietor: **Vayyar Imaging Ltd.**
**56216 Yehud (IL)**

(72) Inventors:
- **LOMNITZ, Yuval**
  **Herzelia 4624093 (IL)**
- **MELAMED, Raviv**
  **7406527 Nes Ziona (IL)**
- **MOSHE, Shay**
  **4922351 Petah-Tikva (IL)**

(74) Representative: **Pearl Cohen Zedek Latzer Baratz UK LLP**
**16 Upper Woburn Place**
**London WC1H 0BS (GB)**

(56) References cited:
**US-A1- 2003 088 180    US-A1- 2006 058 606**
**US-A1- 2008 071 169    US-A1- 2009 129 646**
**US-A1- 2010 069 744    US-A1- 2010 277 184**
**US-A1- 2011 022 325    US-A1- 2011 237 939**

- **B Maklad ET AL: "NEIGHBORHOOD-BASED ALGORITHM TO FACILI- TATE THE REDUCTION OF SKIN REFLECTIONS IN RADAR-BASED MICROWAVE IMAGING", Progress In Electromagnetics Research B, 1 January 2012 (2012-01-01), pages 115-139, XP055315969, Retrieved from the Internet: URL:http://www.jpier.org/PIERB/pierb39/06. 11122208.pdf**
- **O'HALLORAN M ET AL: "Quasi-Multistatic MIST Beamforming for the Early Detection of Breast Cancer", IEEE TRANSACTIONS ON BIOMEDICAL ENGINEERING, IEEE SERVICE CENTER, PISCATAWAY, NJ, USA, vol. 57, no. 4, 1 April 2010 (2010-04-01), pages 830-840, XP011343199, ISSN: 0018-9294, DOI: 10.1109/TBME.2009.2016392**
- **BOND E J ET AL: "Microwave imaging via space-time beamforming for early detection of breast cancer", IEEE TRANSACTIONS ON ANTENNAS AND PROPAGATION, IEEE SERVICE CENTER, PISCATAWAY, NJ, US, vol. 51, no. 8, 1 August 2003 (2003-08-01) , pages 1690-1705, XP011099498, ISSN: 0018-926X, DOI: 10.1109/TAP.2003.815446**

**Description**

**BACKGROUND**

Current popular medical imaging techniques include X-ray imaging (such as Computerized Tomography and mammography), ultrasonic imaging, and MRI (Magnetic Resonance Imaging). Since the 1980s, the use of microwave imaging has been discussed for mapping the interior of the human body and detecting anomalies such as malignant tumors.

Microwave imaging of the human body has developed significantly over the years. Breast imaging has been a popular potential application, both in view of its medical and social importance, and in view of the relatively low-loss materials of which a woman's breast is composed. Typically, a wide band signal is used to sample the transfer function between pairs of antennas over a large frequency range. The frequency range determines the resolution and penetration capabilities. Regardless of the waveforms used for measurement, it is assumed that the reflections (impulse response) between antenna pairs can be estimated, and are referred to herein as the "signals".

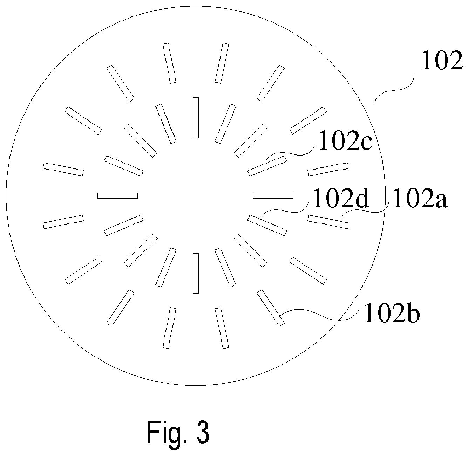

The antenna array is assumed to be a static array containing a large (e.g. several 10-s) number of static antennas. Such an array is depicted in Fig.3.

Microwave-imaging is hindered by the need to identify in-depth features in the human body through the outer attenuating body layers. The faint signal variations caused by in-depth features are masked by reflections from the antennas themselves and the tails of reflections from closer features, such as the interface with the skin.

Multiple algorithms are known for the removal of skin artifacts. The prevailing method, considered here as baseline, is based on subtracting from each of the recorded signals from taking a weighted average of signals recorded at different locations (which are assumed to include similar reflections from the skin). Some current methods are based on subtracting two measurements of the signals, and rotating the array between the two measurements, thus removing any constant factor (such as direct antenna leakage and skin reflection) from the signals.

However these methods suffer from several major drawbacks: first, to obtain symmetrical signals it must be either assumed that the antennas are identical, or the same antenna must be used (i.e. the object or the array must be physically rotated). In practice, sufficiently identical antennas are hard to manufacture, and slight differences between antennas, cables or transceivers may result in significant degradation in skin artifact removal. Rotation of the array or the patient results in mechanical complexity, and especially for breast imaging, it is difficult to ensure the breast would remain exactly in the same position. Therefore, it is desired to remove the skin clutter without assuming the antennas are identical, and without rotating the array.

Second, these cancellations, while reducing clutter, also degrade the reconstructed image. Particularly, if the tumor produces a similar response in neighboring antennas, it would also be cancelled out fully or partially. These effects produce dark spots in the reconstructed image. For example, a differential rotation method also removes targets that are close to the central axis of the array. It is desired to avoid these obstructions as much as possible.

Third, skin cancellation generates artifacts, usually in the form of replicated targets. As an example, the differential rotation method replicates each target, so the image is an overlay of two rotated images. It is desired to avoid these effects as much as possible. Attention is also drawn to US20080071169A1 which discloses various methods and apparatus for reducing signal artifacts resulting from reflections from the surface of an object, US20110022325A1 which discloses a method of measuring the contents of a search volume, 'Neighbourhood-based algorithm to facilitate the reduction of skin reflections in radar-based microwave imaging' by B Malkad et al., 'Quasi-multistatic MIST beamforming for the early detection of breast cancer' by M O'Halloran et al., 'Microwave Imaging via Space-Time Beamforming for Early Detection of Breast Cancer' by E J Bond et al., US20030088180A1 which concerns microwave imaging via space-time beamforming, US20100069744A1 which discloses an imaging system for generating a three-dimensional image of a body part of a patient, US20060058606A1 which concerns microwave examination of individuals carried out by transmitting microwave signals from multiple antenna locations to an individual and receiving the backscattered microwave signals at multiple antenna locations and US20090129646A1 which discloses a method for imaging includes directing a plurality of radio frequency (RF) beams toward a target organ from a plurality of angles.

**SUMMARY**

The invention is defined by the claims. Embodiments of the invention provide a microwave imaging sensor with increased robustness to skin reflection, which does not require a physical rotation of the array or the antennas.

In particular, a method is provided for enhancing microwave imaging of an object using an array of antennas having transmit-receive, the method comprising: collecting microwave responses for multiple combinations of transmit antennas and receive antennas of the array; determining a plurality of reference pairs for at least one pair of transmit antenna and receive antennas, wherein a reference pair is selected from a group consisting of: a set of pairs which are rotations of each other; and a set of neighboring pairs; performing, by a data processing unit, an estimation of skin reflection, where the estimation for an antenna pair is based on weighted signals received from another antenna pair during a measurement, wherein the other antenna pair is selected from the plurality of reference pairs for the antenna pair, and wherein a weight for the weighted

signals includes a frequency domain response unique to the antenna pair, to account for antenna pairs and respective transmit-receive paths thereof which are not identical;

The methods disclosed for embodiments of the invention can apply separately to other imaging techniques or other skin artifact removal techniques; For example, an improved imaging algorithm disclosed herein may be used in conjunction with physical rotation. Furthermore, the methods disclosed herein may be applied with variations to other radio or sonar imaging problems where it is desired to remove a constant background effect with minimum effect on the image.

Therefore, according to an embodiment of the present invention, there is provided a method for enhancing microwave imaging of an object, including: (a) collecting microwave responses for multiple combinations of transmit antennas and receive antennas; (b) performing an estimation skin reflection, where the estimation for an antenna pair is based on signals received from or signals recorded at another antenna pair during a measurement; (c) performing a cancellation of the skin reflection based on the estimation; and (d) generating an image from the corrected signals after the cancellation.

Figure 2 shows a MIMO microwave-imaging system applied to imaging of a woman's breast, according to an embodiment of the present invention;

Figure 3 illustrates an example of a prior-art spherical antenna array.

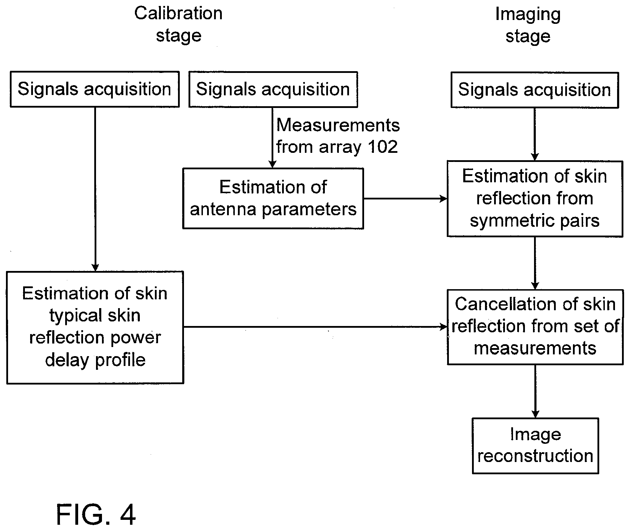

Figure 4 is a flowchart of a method according to an embodiment of the invention.

Figure 5 is a microwave image of a breast.

Figure 6 is a three-dimensional depiction of the results of microwave imaging of a breast.

For addition simplicity and clarity of illustration, elements shown in the figures are not necessarily drawn to scale, and the dimensions of some elements may be exaggerated relative to other elements. In addition, reference numerals may be repeated among the figures to indicate corresponding or analogous elements.

## DETAILED DESCRIPTION

Exemplary embodiments of the invention are described below. Those skilled in the art will appreciate that various components, calculations, operations, etc may be changed while keeping the main functions described. The application of the invention is not limited to the demonstrative embodiments described below.

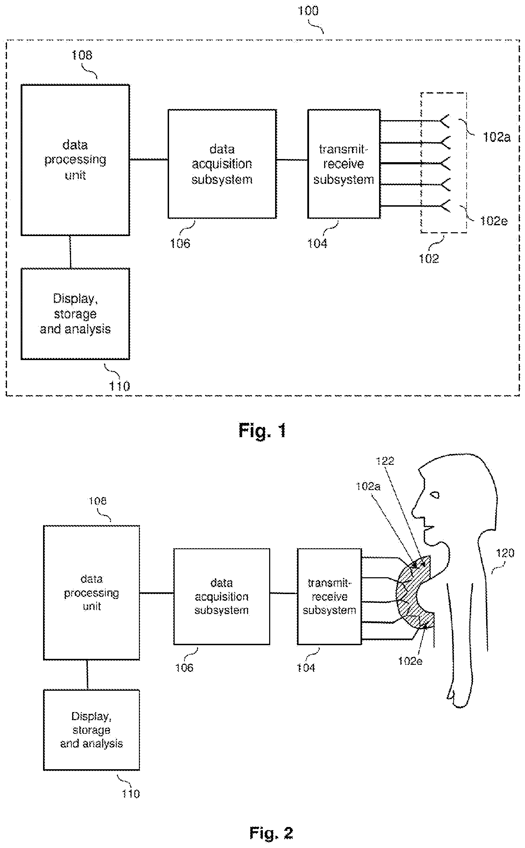

In an embodiment of the invention, depicted in Figure 1, a "MIMO radar" system **100** is composed of an antenna array **102,** a transmit-receive subsystem **104,** a data acquisition subsystem **106,** a data processing unit **108,** and a console **110.**

The antenna array is composed of multiple antennas **102a-102e,** typically between few and few tens (for example 30) antennas. The antennas can be of many types known in the art, such as printed antennas, waveguide antennas, dipole antennas or "Vivaldi" broadband antennas. The antenna array can be linear or two-dimensional, flat or conformal to the region of interest.

The transmit-receive subsystem **104** is responsible for generation of the microwave signals, coupling them to the antennas **102a-102e,** reception of the microwave signals from the antennas and converting them into a form suitable for acquisition. The signals can be pulse signals, stepped-frequency signals and the like. The generation circuitry can involve oscillators, synthesizers, mixers, or it can be based on pulse oriented circuits such as logic gates or step-recovery diodes. The conversion process can include down conversion, sampling, and the like. The conversion process typically includes averaging in the form of low-pass filtering, to improve the signal-to-noise ratios and to allow for lower sampling rates. The transmit-receive subsystem can perform transmission and reception with multiple antennas at a time or select one transmit and one receive antenna at a time, according to a tradeoff between complexity and acquisition time.

The data acquisition subsystem **106** collects and digitizes the signals from the transmit-receive subsystem while tagging the signals according to the antenna combination used and the time at which the signals were collected. The data acquisition subsystem will typically include analog-to-digital (A/D) converters and data buffers, but it may include additional functions such as signal averaging, correlation of waveforms with templates or converting signals between frequency and time domain.

The data processing unit **108** is responsible for converting the collected signals into responses characterizing the medium under test, and performing the algorithms for converting the sets of responses into image data. In the context of the invention described herein, this unit is responsible for the skin clutter cancellation. The data processing unit is usually implemented as a high-performance computing platform, based either on dedicated Digital Signal Processing (DSP) units, general purpose CPUs, or, according to newer trends, Graphical Processing Units (GPU).

A final step in the process is making use of the resulting image, either in the form of visualization, display, storage, archiving, or input to feature detection algorithms. This step is exemplified in Fig. 1 as console **110**. The console is typically implemented as a general purpose computer with appropriate application software. According to system type, the computer can be stationary, laptop, tablet, palm or industrial ruggedized computer. It should be understood that while Figure 1 illustrates functional decomposition into processing stages, some of those can be implemented on the same hardware (such as a common processing unit) or distributed over multiple and even remote pieces of hardware (such as in the case of multiprocessing or cloud computing).

Figure 2 illustrates application of the Doppler assisted MIMO radar system to the examination of a woman's breast. In this illustration, the antenna array **102** is coupled to the breast of the subject **120**. The antennas **102a-102e** of the array **102** are situated in a conformal cup-like shape, shown from a top view in Figure 3, and an intermediate medium **122** is used to create improved electromagnetic coupling between the antenna radiation and the breast. The purpose of the MIMO radar system in such application is typically to search for malignant tumors.

The system operation is generally as follows. At each time, the microwave transceiver transmits a predesigned signal from one or more of the antennas, and receives the signal from one or more other antennas. When the system is used for human body visualization, the signals typically occupy frequencies between about 10 MHz and 10Ghz. Particular popularity and attention has recently been drawn to the 3.1-10.6 GHz range, which allows license-exempt ultra-wideband (UWB) operation at low signal levels. There is an advantage to using lower frequencies in view of better penetration into the human body, but also to higher frequencies, in view of shorter wavelength and better spatial resolution. Use of a wide frequency range allows high temporal resolution, facilitating discrimination of features according to their depth (distance from the antennas). There is a variety of choices in selecting signals for microwave imaging applications, such as frequency-swept waveforms and pulse waveforms. By one or more such transmissions, the transfer function of the medium between the transmit antennas and receive antennas is estimated. The processing unit then processes these signals to generate an image.

The image reconstruction algorithms usually start with a collection of responses $y_{ij}(t)$ denoting the impulse response between antenna $i$ and antenna $j$ at time $t$. The estimation of the transfer functions $y_{ij}(t)$ involves calibration processes known in the art.

A known algorithm for reconstructing an image from the impulse responses of the medium is called "Delay and Sum" (DAS), and will be used here as a reference. For each point $r$ in some designated volume in the three dimensional space, and for each antenna pair (from antenna $i$ to antenna $j$) the expected delay from antenna $i$ to point $r$ and back to antenna $j$ is calculated, considering the propagation velocity through the medium (which is assumed to have known electrical properties). Denote this delay by $T_{ij}(r)$. Then the reconstructed image at location $r$ is created by summing the estimated impulse responses $y_{ij}(t)$ of each pair $i,j$ at the expected delay $T_{ij}(r)$, i.e.

$$I_{DAS}(r) = \sum_{ij} y_{ij}(T_{ij}(r)) \tag{1}$$

where the summation is over all antenna pairs. In some embodiments, a function of $I_{DAS}(r)$ such as its absolute or power is presented as the image. Assuming a reflector exists at point r then we expect a positive pulse to exist at position $T_{ij}(r)$ in all, or most, pairs, creating high intensity of the reconstructed image at this point. This algorithm, and variations of it, are well known in the art. In this algorithm, the responses $y_{ij}(t)$ are assumed to be identical and perfect pulses. Calibration of the antennas, cables and measurement equipment is applied to the recorded signals in order to produce $y_{ij}(t)$, so this assumption holds within some approximation.

For each pair $ij$ let $R_{ij}$ be a group of "reference pairs" for the pair $ij$, including the pair $ij$ itself. Depending on the setup, these pairs may be the set of all pairs which are rotations of each other (i.e. are arrayed in a circle), or a set of neighboring pairs. For example, referring to Figure 3, the pair **102a-102c** is a rotation of the pair **102b-102d** and therefore each pair may be used in the reference group of the other. It is assumed that the skin reflection signal is similar between these pairs. That is, the signal model is:

$$Y_{mn}(f) = \underbrace{A_{mn}(f) \cdot S_{ij}(f)}_{s'_{ij}(f)} + \tilde{Y}_{mn}(f), \qquad \forall (m,n) \in R_{ij} \tag{2}$$

where $Y_{mn}(f)$ is the frequency domain (fourier transform) signal of $y_{mn}(t)$, $A_{mn}(f)$ is the frequency domain response unique to the specific pair, $S_{ij}(f)$ is the common reflection from the skin which is common in the group, and $\tilde{Y}_{mn}(f)$ is the desired signal from the target, not including the skin reflection, and is potentially considerably weaker than $Y_{mn}(f)$. The relation is assumed to hold for all the pairs in $R_{ij}$.

According to the invention, the inclusion of the response $A_{mn}(f)$ manifests the fact that the antenna pairs, including their respective transmit and receive paths are not identical. The difference may stem from a difference in antennas, reflections from the antenna, cables, switches, and the transmit and receive chains. Due to this factor, which inserts time spread, simple linear subtraction is limited in its performance.

Supposing the responses $A_{mn}(f)$ were estimated, the estimate of the skin reflection component $S'_{ij}(f)$ in the signal $Y_{ij}(f)$ is

$$\hat{S}'_{ij}(f) = A_{ij}(f) \cdot \frac{\sum_{(m,n) \in R_{ij}} A^*_{mn}(f) \cdot Y_{mn}(f)}{\sum_{(m,n) \in R_{ij}} |A_{mn}(f)|^2} = \sum_{(m,n) \in R_{ij}} w_{mn}(f) \cdot Y_{mn}(f) \tag{3}$$

In other words, the common signal $S(f)$ is estimated from $Y_{mn}(f)$, $(m, n) \in R_{ij}$ by averaging with complex factors per frequency, and then multiplied by the pair's response. The estimate Eq.(3) is indifferent to multiplication of all $A_{mn}(f)$ by a frequency dependent constant, as such will be cancelled out in the nominator and denominator.

In some embodiments of the invention, the responses $A_{mn}(f)$ are estimated by using a known reflector, such as a metal cup, positioned at the same location as the skin. In this case $\tilde{Y}_{mn}(f) = 0$ and knowledge of $S(f)$ is not required due to the aforementioned indifference property, thus $A_{mn}(f)$ can be set equal to the measured signals in this calibration measurement.

In other embodiments of the invention, the responses $A_{mn}(f)$ are estimated from a multitude of measurements, $Y^k_{ij}(f)$ taken with different materials or different patients (where $k$ denotes the index of the measurement, i.e. a different subject or phantom). In this case, $Y^k_{mn}(f) \approx A_{mn}(f) \cdot S_k(f)$, i.e. the antenna pair response is constant over measurements, and the skin reflection is constant over antenna pairs. The coefficients $A_{mn}(f)$ are estimated (up to a factor), for each value of $f$ separately, by applying Singular Value Decomposition (SVD) to the matrix $Y_f$ with elements $Y^k_{mn}(f)$, where $k$ comprises the column index and each $(m, n)$ is assigned a column (i.e. the pair translates to a row index), and taking the first (most substantial) singular vector.

Those skilled in the art will appreciate that this method is easily generalized for treating multiple background reflectors having different responses and different directions (and hence potentially different $A$-s), or secondary reflections from the skin (due to resonance), by taking several singular vectors rather than one.

In yet other embodiments of the invention, the complex weights $w_{mn}(f)$ of Eq.(3) are directly estimated from a multitude of measurements by finding such weights that minimize the total energy of $\tilde{Y}_{ij}(f) = Y_{ij}(f) - \hat{S}'_{ij}(f)$ (where $\hat{S}'_{ij}(f)$ is substituted with the second form of Eq.(3)) in a designated window. In this embodiment, the number of reference measurements must be larger than the number of pairs in the reference set $R_{ij}$ in order to obtain meaningful results.

In some embodiments of the invention, the signal model (Eq.(2)) includes a full or partial scattering parameters model (S-Parameters) of the medium and the antennas. I.e. it is assumed that the S-parameters of the antennas are different between antennas and constant over time, while the S-Parameters of the skin are constant over different antenna pairs. The values $S_{mn}$ and $A_{mn}$ in Eq.(3) above are replaced with their respective S-parameter models, and $A_{nm}$ is estimated from one or more calibration recordings.

According to various embodiments of the invention, online filtering of the set of reference pairs $R_{ij}$ as a function of the signals may be applied prior to applying Eq.(3). For each time window of a predetermined size, a set of outliers of $y_{mn}(t)$ over this window is calculated and removed from the set, before continuing to apply Eq.(3). The outliers may be detected as the signals with largest Euclidian distance from the mean signal $\sum_{mn \in R_{ij}} w_{mn}(f) Y_{mn}(f)$ after converting to time domain, taken over the time window. This method further decreases the effect of the targets themselves on the skin subtraction, as target would typically appear at different time windows in each of the measurements, and would be rejected as outliers.

According to some embodiments of the invention, additional weights are placed inside the sum in Eq.(3), to account for correlation of the skin reflection between different pairs. As an example, pairs *mn* which are farther apart from the pair *ij* would typically have a smaller weight than pairs that are near the pair *ij*.

For each pair of antennas, let $P_{ij}(\tau)$ reflect the power-delay profile of the skin reflection, i.e. this factor is proportional to the typical amount of energy that is expected to exist in a small time window around delay $\tau$. In some embodiments of the invention, $P_{ij}(\tau)$ may be measured from a multitude of recordings, while in others, a simple model, such as exponential decay $P_{ij}(\tau) = \alpha_{ij}e^{-\tau d_{ij}}Ind(\tau \geq T_{ij})$ is assumed, where the parameters $\alpha_{ij}$, $d_{ij}$, $T_{ij}$ are derived based on various considerations. This delay profile would usually be equal for different pairs in the symmetric reference group but different between different groups (e.g. for antennas that are farther apart, the reflection would be weaker and delayed).

The corrected signals after skin cancellation are calculated as:

$$\tilde{y}_{ij}(t) = y_{ij}(t) - \frac{P_{ij}(t)}{P_{ij}(t) + \lambda} \cdot \hat{s}'_{ij}(t) \qquad (4)$$

Where $\hat{s}'_{ij}(t)$ is the time domain representation of the skin reflection estimate $\hat{S}'_{ij}(f)$ found in Eq.(3), and $\lambda$ is a constant. In some embodiments of the invention, $\lambda$ is selected proportional to the estimated noise variance and inversely proportional to the number of pairs in $R_{ij}$. Notice that while perfect cancellation would, for example, cancel a signal from the center of the array in case of rotational symmetry, the soft cancellation of Eq.(4) would typically leave the center of the array intact. This is because for some pairs, the skin reflection would be either far in time from the delay that characterizes the center of the array, while for others, it may be close in time but weaker in amplitude.

In some embodiments of the invention, polarization is used to reduce the reflection from the skin; for example, adjacent antennas are of different polarization, or cross-polarized antennas are used. In these embodiments, the power of the skin reflection is in general smaller for cross-polarized antenna pairs, and therefore the values of $P_{ij}(t)$ associated those pairs is allowed, and the coefficient in Eq.(4) would give a lower weight to subtracting the skin effect.

In some embodiments of the invention, tuning parameters may be added to the various elements in equations (3),(4). In one embodiment, a variable time shift and gain is added to measurements $Y_{ij}^k(f)$ taken from other tests in order to compensate for differences due to temperature, physical shifts, the measurement equipment, etc, and these parameters are tuned to obtain best match with the measured signal (according to the criterions specified above); likewise, in some embodiments of the invention, tuning parameters are added to $\hat{s}'_{ij}(t)$ in Eq.(4) and are determined by minimizing the energy of $\tilde{y}_{ij}(t)$.

Because Eq.(3)-(4) comprise a spatial-temporal filter, the straightforward application of DAS Eq. (1) to the skin corrected signals $\tilde{y}_{ij}(t)$ as considered in existing art produces additional false targets on the reconstructed image $I_{DAS}(r)$. A simple example for the purpose of clarification is physical rotation or differential imaging in which $\tilde{y}_{ij}(t)$ is effectively $\tilde{y}_{ij}(t) = y_{ij}(t) - y_{(ij)+ofs}(t)$, where $(ij) + ofs$ denotes a pair which is at a given rotational offset from the pair *ij* (ignoring the fact the two elements were measured at different times), and as a result each target would appear twice in the reconstructed image.

In some embodiments of the invention, the above effect is minimized by applying a correcting spatial-temporal filter to the corrected measurements $\tilde{y}_{ij}(t)$ as follows:

$$I(r) = \sum_{ij} \sum_{(mn) \in R_{ij}} \left[ q_{m,n,i,j}^{(r)}(t) * \tilde{y}_{mn}(t) \right]_{t=T_{mn}(r)} \qquad (5)$$

Where $q_{m,n,i,j}^{(r)}(t)$ is a set of filters which may vary (in general) as a function of the pairs and the position in space, and "*" denotes time domain convolution. In other words, the signals of pairs *mn* that participate in the skin reflection correction for the pair *ij*, are taken at their hypothesized target location *r*, and linearly weighted. Notice that the sum over *mn* includes also the component *ij* which would typically have the most significant weight. Notice that unlike Eq.(3)-(4) where symmetric pairs are considered at the same time point $t = T_{ij}(r)$, here, the signal of each pair is taken at the point where a target at location $r$ would appear at that pair.

The coefficients $q_{m,n,i,j}^{(r)}(t)$ of the spatial-temporal filter can be obtained via various criteria. The combined effect of the skin removal stage (Eq(3)-(4)) and imaging via Eq.(5) for a given target at position $r$ can be calculated. Then $q_{m,n,i,j}^{(r)}(t)$ can be determined so as to minimize a quality criterion, related to the total noise and artifacts (side lobes or secondary images). In some embodiments, $q_{m,n,i,j}^{(r)}(t)$ are determined so as to minimize a weighted sum of the noise and the average sidelobe energy. In other embodiments, $q_{m,n,i,j}^{(r)}(t)$ are determined so as to maximize the peak to side-lobe ratio, between the value of $I(r)$ at the target, and the value of the strongest artifact

For the purpose of illustration, in the simple example of differential imaging $\tilde{y}_{ij}(t) = y_{ij}(t) - y_{(ij)+ofs}(t)$, the spatial filter can be chosen as $q_{m,n,i,j}^{(r)}(t) = \sum_k \alpha_k \cdot \delta_{mn} = {}_{(ij)+k \cdot ofs} \cdot \delta(t)$. I.e. no temporal filtering is applied, and summation of pairs in the symmetry group with different weights is used. The effect is that the spatial spreading pattern of a target is determined by the convolution of $a_k$ with the filter $[1,-1]$ generated by differential imaging. Choosing the simple filter $a_0 = 1$, $\alpha_1 = -1$ (and all rest are 0) yields target to artifact ratio of 6dB where the original DAS Eq.(1) yields 0dB, and the filter $a_k = \max(1 - \beta(k - 0.5), 0) \cdot sign(k - 0.5)$, with $\beta \le 1$ of choice, yields peak to average of $20 \cdot \log_{10}\left(\frac{4-2\beta}{\beta}\right) \mathrm{dB}.$

**Methods for Detecting Cancer**

One embodiment of the invention provides a method of detecting or locating cancer in a tissue of a subject comprising the step of: contacting the tissue of the subject with an apparatus for enhanced microwave imaging, wherein the apparatus includes microwave transmit antennas, microwave receive antennas, and a means for collecting microwave responses from multiple transmit and receive antennas.

Another embodiment further provides collecting microwave responses for multiple combinations of transmit antennas and receive antennas.

A further embodiment provides estimating skin reflection, where the estimation for an antenna pair is based on signals received from or signals recorded at other antenna pairs during the same measurement and used as reference, wherein the estimation is performed according to Eq.(3). A related embodiment provides performing cancellation of the skin reflection based on the estimation. Another related embodiment generates an image from the corrected signals after the cancellation.

Additional embodiments provide apparatus for the detection of a precancerous or cancerous condition in a breast. In one related embodiment, the apparatus includes a computer for digitizing mammogram image data. In one such embodiment, the apparatus includes a computer for recording microwave responses from multiple transmit and receive antennas.

A further embodiment of the invention provides a computer product including a computer-readable tangible storage medium containing non-transitory executable instructions for a computer to perform methods disclosed herein, or variations thereof.

In an additional embodiment of the present invention, the apparatus detects a pathological disorder, such as a cancer or a tumor.

In yet additional embodiments of the present invention, the apparatus is used for detecting a carcinoma, sarcoma, lymphoma, blastoma, glioblastoma, or melanoma. In another such embodiment, the tumor detected includes tumors of the brain, esophagus, nose, mouth, throat, lymphatic system, lung, breast, bone, liver, kidney, prostate, cervix, head or neck, skin, stomach, intestines, pancreas, or combinations thereof. Further embodiments of the invention are used to detect breast cancer and prostate cancer.

Other embodiments of the invention provide apparatus for detecting precancerous conditions, such as benign prostatic hyperplasia (BPH), actinic keratosis, Barrett's esophagus, atrophic gastritis, cervical dysplasia, and precancerous breast lesions.

According to certain embodiments of the invention, the configuration and/or shape of the apparatus is adjusted to conform with the shape of the body at the point at which the apparatus is attached, as shown in Figure 2 for the breast, and as would be clear to someone familiar with the field. In a related embodiment, the apparatus further includes a component for securing tissue in a fixed position, and to prevent tissue from moving during diagnosis.

**EXAMPLE of Detecting Breast Cancer**

**Materials and Methods**

**Human Subjects**

Three groups are studied: women with breast cancer found in a breast biopsy, women with no histologic evidence of breast cancer in a breast biopsy, and healthy volunteers. The first two groups include women undergoing open surgical biopsy to exclude malignancy. Examination prior to surgery includes a physical examination, mammogram, and additional breast imaging where clinically indicated. Subjects are women 18 years of age and older with no history of previously-diagnosed cancer at any site. The third group includes healthy age-matched volunteers who are recruited from members of the general population with no history of cancer or other chronic disease. The institutional review boards of all participating institutions approve the research.

**Detection of Breast Cancer**

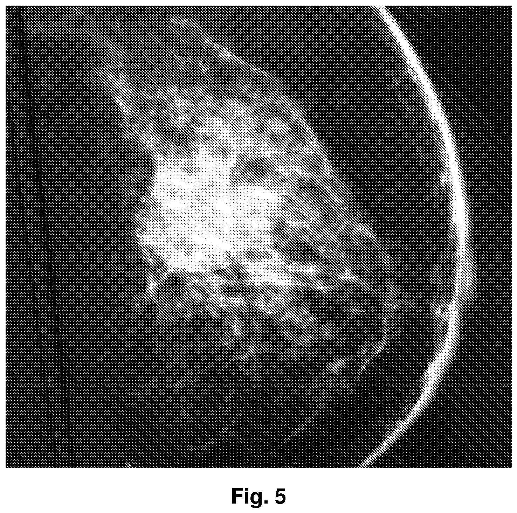

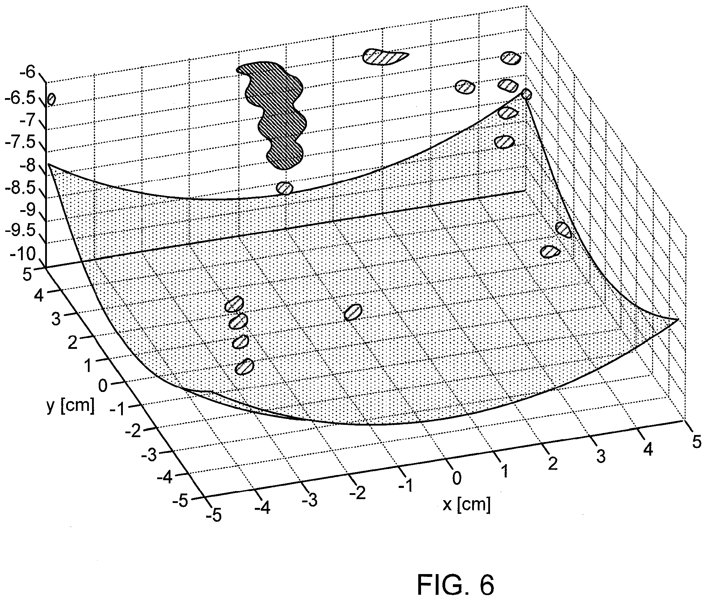

Subjects are microwave-imaged for detecting breast cancer using a microwave imaging sensor as described herein as well as using previously known microwave imaging sensors (Figs. 5-6). In addition, subjects underwent X-ray mammography as a further control. All biopsy slides are independently reviewed by two pathologists and assessed according to standard criteria for breast cancer. Discordant readings are excluded from the data analysis.

**Results**

The apparatus for detecting breast cancer according to embodiments of the present invention as disclosed herein provides extremely high accuracy of breast cancer detection compared to other microwave techniques and comparable to standard X-ray mammography, with very few false positives. Biopsy results are used to confirm the diagnosis of each patient to determine the accuracy of the detection apparatus of the present invention compared to other microwave techniques and standard mammography.

It is also understood that the above-disclosed embodiments are non-limiting and exemplary, and that different additional embodiments of the present invention have different antenna arrays, clutter characteristics and operate with different reconstruction algorithms.

**Claims**

**1.** A method for enhancing microwave imaging of an object using an array of antennas (102) having transmit-receive, the method comprising:

collecting microwave responses for multiple combinations of transmit antennas and receive antennas of the array (104);
determining a plurality of reference pairs for at least one pair of transmit antenna and receive antennas, wherein a reference pair is selected from a group consisting of:

a set of pairs which are rotations of each other; and
a set of neighboring pairs;

performing, by a data processing unit (108), an estimation of skin reflection;
performing, by the data processing unit, a cancellation of the skin reflection based on the estimation; and
generating by the data processing unit an image from the corrected signals after the cancellation;
**characterized in that**
the estimation of skin reflection for an antenna pair is based on weighted signals received from another antenna pair during a measurement,
wherein the other antenna pair is selected from the plurality of reference pairs for the antenna pair, and
wherein a weight for the weighted signals includes a frequency domain response unique to the antenna pair, to account for antenna pairs and respective transmit-receive paths thereof which are not identical.

**2.** The method of claim 1, wherein weights for the weighted signals are learned from multiple recordings by finding weights which yield a best match between signals acquired in the recordings.

3. The method of claim 1, wherein a weight for the weighted signals is learned from a set of multiple signals recorded with various materials or subjects, by applying singular value decomposition (SVD) to a matrix generated from responses measured at a single frequency for antenna pairs in a reference group.

4. The method of claim 1 wherein the cancellation comprises attenuating the estimation before subtracting the estimation from the measurement.

5. The method of claim 4, wherein a weight for an attenuation depends on a location in a reconstructed image.

6. The method of claim 4, wherein a weight for an attenuation is different for different polarization states of the antenna pair.

7. The method of claim 1 wherein the imaging algorithm performs a spatial-temporal filtering on the signals after the cancellation.

8. A method of detecting and locating a cancer in a tissue of a subject comprising:

contacting the tissue of the subject with an apparatus for enhanced microwave imaging, wherein the apparatus comprises microwave transmit antennas (102), microwave receive antennas, and a means for collecting microwave responses for multiple transmit and receive antennas (106); and
performing the steps of the method of claim 1.

9. The method of claim 8, wherein a weight used for cancellation is a function of the parameters of an individual antenna pair.

10. The method of claim 9, wherein the weight is learned from multiple recordings by finding weights which yield a best match between signals acquired in the recordings.

11. The method of claim 9, wherein the weight is learned from a set of multiple signals recorded with various subjects, by applying a singular value decomposition (SVD) to a matrix generated from a response measured at a single frequency for an antenna pair.

12. The method of claim 8, wherein the estimation is attenuated before being subtracted from the measurement.

13. The method of claim 12, wherein a weight for an attenuation depends on a location in a reconstructed image.

14. The method of claim 12, wherein a weight for an attenuation is different for different polarization states of the antenna pair.

15. The method of claim 8, where the imaging algorithm performs a spatial-temporal filtering on the signals after the cancellation.


**Patentansprüche**

1. Verfahren zur Verbesserung von Mikrowellen-Bildgebung eines Objekts unter Verwendung einer Anordnung von Sende-Empfangs-Antennen (102), wobei das Verfahren Folgendes umfasst:

Erheben von Mikrowellenantworten für mehrere Kombinationen von Sendeantennen und Empfangsantennen der Anordnung (104);
Bestimmen einer Vielzahl von Referenzpaaren für mindestens ein Paar Sendeantennen und Empfangsantennen, wobei ein Referenzpaar von einer aus Folgendem bestehenden Gruppe ausgewählt wird:

Einem Satz Paare, die Drehungen voneinander sind; und
Einen Satz benachbarter Paare;

Durchführen einer Schätzung einer Hautreflexion mithilfe einer Datenverarbeitungseinheit (108);
Durchführen einer Annullierung der Hautreflexion aufgrund der Schätzung mithilfe einer Datenverarbeitungseinheit; und

Erzeugen eines Abbildes von den korrigierten Signalen nach der Annullierung mithilfe einer Datenverarbeitungseinheit;

**dadurch gekennzeichnet, dass**

die Schätzung der Hautreflexion für ein Antennenpaar auf gewichteten Signalen, die von einem anderen Antennenpaar während einer Messung empfangen wurden, basiert,

wobei das andere Antennenpaar von einer Vielzahl von Referenzpaaren für das Antennenpaar ausgewählt wird, und

wobei eine Gewichtung für das gewichtete Signal eine Frequenzdomänenantwort beinhaltet, die für das Antennenpaar eindeutig ist, um Antennenpaare und die jeweiligen Sende-Empfangswege davon zu erfassen, die nicht identisch sind.

2. Verfahren nach Anspruch 1, wobei Gewichtungen für die gewichteten Signale von mehreren Aufnahmen in Erfahrung gebracht werden, indem Gewichtungen gefunden werden, die die beste Übereinstimmung zwischen in Aufnahmen erlangten Signalen ergeben.

3. Verfahren nach Anspruch 1, wobei eine Gewichtung für die gewichteten Signale von einem Satz mehrerer Signale, die mit verschiedenen Materialien oder Subjekten aufgezeichnet wurden, in Erfahrung gebracht wird, indem eine Singulärwertzerlegung ("singular value decomposition", SVD) auf eine Matrix, die von gemessenen Antworten bei einer Einzelfrequenz für Antennenpaare in einer Referenzgruppe erzeugt wird, angewendet wird.

4. Verfahren nach Anspruch 1, wobei die Annullierung ein Dämpfen der Schätzung umfasst, bevor die Schätzung von der Messung subtrahiert wird.

5. Verfahren nach Anspruch 4, wobei eine Gewichtung für eine Dämpfung von einem Ort in einem rekonstruierten Bild abhängig ist.

6. Verfahren nach Anspruch 4, wobei eine Gewichtung für eine Dämpfung für verschiedene Polarisierungszustände des Antennenpaares unterschiedlich ist.

7. Verfahren nach Anspruch 1, wobei der Bildgebungsalgorithmus nach der Annullierung eine Raum-Zeit-Filterung an den Signalen vornimmt.

8. Verfahren zum Erfassen und Lokalisieren einer Krebserkrankung bei einem Subjekt, das Folgendes umfasst:

Kontaktieren des Gewebes des Subjekts mit einer Vorrichtung zur besseren Mikrowellenbildgebung, wobei die Vorrichtung Mikrowellen-Sendeantennen (102), Mikrowellen-Empfangsantennen und ein Mittel zum Erheben von Mikrowellenantworten auf mehrere Sende- und Empfangsantennen (106) umfasst; und

Durchführen der Verfahrensschritte nach Anspruch 1.

9. Verfahren nach Anspruch 8, wobei eine für eine Annullierung verwendete Gewichtung von den Parametern eines individuellen Antennenpaares abhängig ist.

10. Verfahren nach Anspruch 9, wobei die Gewichtung aufgrund mehrerer Aufnahmen in Erfahrung gebracht wird, indem Gewichtungen gefunden werden, die die beste Übereinstimmung der in den Aufnahmen erlangten Signale ergeben.

11. Verfahren nach Anspruch 9, wobei die Gewichtung aufgrund eines Satzes mehrerer bei verschiedenen Subjekten aufgenommener Signale in Erfahrung gebracht wird, indem eine Singulärwertzerlegung (SVD) auf eine Matrix, die von einer bei einer Einzelfrequenz gemessenen Antwort für ein Antennenpaar erzeugt wird, angewendet wird.

12. Verfahren nach Anspruch 8, wobei die Schätzung gedämpft wird, bevor sie von der Messung subtrahiert wird.

13. Verfahren nach Anspruch 12, wobei eine Gewichtung für eine Dämpfung von einem Ort in einem rekonstruierten Bild abhängig ist.

14. Verfahren nach Anspruch 12, wobei eine Gewichtung für eine Dämpfung in Bezug auf verschiedene Polarisationszustände des Antennenpaars unterschiedlich ist.

**15.** Verfahren nach Anspruch 8, wobei der Bildgebungsalgorithmus nach der Annullierung eine Raum-Zeit-Filterung an den Signalen vornimmt.

**Revendications**

**1.** Procédé pour améliorer l'imagerie micro-onde d'un objet utilisant un ensemble d'antennes (102) capable de transmettre-recevoir, le procédé comprenant :

la collection de réponses micro-onde provenant de multiples combinaisons d'antennes de transmission et d'antennes de réception de l'éventail de signaux (104) :

la détermination d'une pluralité de paires référentielles pour au moins une paire d'antenne de transmission et d'antennes de réception, dans lequel une paire référentielle est sélectionnée à partir d'un groupe consistant :

d'un ensemble de paires qui sont des rotations l'une de l'autre ; et
d'un ensemble de paires avoisinantes :

la mise en oeuvre, par une unité de traitement de données (108), d'une estimation du réfléchissement de la peau ;
la mise en oeuvre, par l'unité de traitement de données, d'une annulation du réfléchissement de la peau basée sur l'estimation ; et
la génération, par l'unité de traitement de données, d'une image à partir des signaux corrigés après l'annulation ;

**caractérisé en ce que**
l'estimation du réfléchissement de la peau pour une paire d'antennes est basée sur des signaux pondérés reçus d'une autre paire d'antennes au cours d'une mesure,
dans lequel l'autre paire d'antennes est sélectionnée parmi la pluralité de paires référentielles pour la paire d'antennes, et
dans lequel une pondération pour les signaux pondérés, inclut une réponse unique de domaine de fréquence à la paire d'antennes, pour prendre en compte les paires d'antennes et leurs chemins respectifs de transmission-réception qui ne sont pas identiques.

**2.** Procédé de la revendication 1, dans lequel les pondérations des signaux pondérés sont apprises à partir d'enregistrements multiples en trouvant les pondérations qui produisent une meilleure correspondance entre les signaux acquis durant les enregistrements.

**3.** Procédé de la revendication 1, dans lequel une pondération pour les signaux pondérés est apprise à partir d'un ensemble de signaux multiples enregistrés avec divers matériaux ou patients, en appliquant une décomposition de valeur singulière (SVD) à une matrice générée à partir de réponses mesurées à une fréquence unique pour des paires d'antennes dans un groupe référentiel.

**4.** Procédé de la revendication 1, dans lequel l'annulation comprend d'atténuer l'estimation avant de soustraire l'estimation de la mesure.

**5.** Procédé de la revendication 4, dans lequel une pondération pour une atténuation dépend d'un lieu dans une image reconstituée.

**6.** Procédé de la revendication 4, dans lequel une pondération pour une atténuation est différente pour différents états de polarisation de la paire d'antennes.

**7.** Procédé de la revendication 1, dans lequel l'algorithme d'imagerie effectue un filtrage spatio-temporel sur les signaux après l'annulation.

**8.** Procédé de détection et de localisation d'un cancer dans un tissu d'un patient comprenant :

le contact du tissu du patient avec un appareil d'imagerie micro-onde amélioré, dans lequel l'appareil comprend des antennes de transmission micro-onde (102), des antennes de réception micro-onde et un moyen pour collecter les réponses micro-onde de multiples antennes de transmission et de réception (106) ; et

la mise en oeuvre des étapes du procédé de la revendication 1.

9. Procédé de la revendication 8, dans lequel une pondération utilisée pour l'annulation est une fonction des paramètres d'une paire d'antennes individuelles.

10. Procédé de la revendication 9, dans lequel la pondération est apprise à partir d'enregistrements multiples en trouvant des pondérations qui produisent une meilleure correspondance entre des signaux acquis durant les enregistrements.

11. Procédé de la revendication 9, dans lequel la pondération est apprise à partir d'un ensemble de multiples signaux enregistrés avec divers patients, en appliquant une décomposition de valeur singulière (SVD) à une matrice générée à partir d'une réponse mesurée à une fréquence unique pour une paire d'antennes.

12. Procédé de la revendication 8, dans lequel l'estimation est atténuée avant d'être soustraite de la mesure.

13. Procédé de la revendication 12, dans lequel une pondération pour une atténuation dépend d'un lieu dans une image reconstruite.

14. Procédé de la revendication 12, dans lequel une pondération est différente pour différents états de polarisation de la paire d'antennes.

15. Procédé de la revendication 8, ou l'algorithme d'imagerie effectue un filtrage spatio-temporel sur les signaux après l'annulation.

Fig. 1

Fig. 2

102

102c

102d 102a

102b

Fig. 3

Calibration
stage

Imaging
stage

| Signals acquisition | Signals acquisition |
|---|---|

Measurements
from array 102

Signals acquisition

Estimation of
antenna parameters

Estimation of skin
reflection from
symmetric pairs

Estimation of skin
typical skin
reflection power
delay profile

Cancellation of skin
reflection from set of
measurements

Image
reconstruction

FIG. 4

**Fig. 5**

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080071169 A1
- US 20110022325 A1
- US 20030088180 A1
- US 20100069744 A1
- US 20060058606 A1
- US 20090129646 A1

**Non-patent literature cited in the description**

- **B MALKAD.** *Neighbourhood-based algorithm to facilitate the reduction of skin reflections in radar-based microwave imaging*
- **M O'HALLORAN.** *Quasi-multistatic MIST beamforming for the early detection of breast cancer*
- **E J BOND.** *Microwave Imaging via Space-Time Beamforming for Early Detection of Breast Cancer*